# EUROPÄISCHE PATENTSCHRIFT

(11) **EP 0 736 535 B1**
(45) Veröffentlichungstag und Bekanntmachung des Hinweises auf die Patenterteilung: **11.11.1998**
(21) Anmeldenummer: 96810198.0
(22) Anmeldetag: 29.03.1996
(51) Int. Cl.: C07D 493/18

(54) **Verfahren zur Herstellung von Dihydroartemisinin-hemisuccinat**
Process for the production of dihydroartemisin-hemisuccinate
Procédé pour la préparation de la hémisuccinate de la dihydroartemisin

(30) Priorität: 03.04.1995 BG 99545/95
(43) Veröffentlichungstag der Anmeldung: 09.10.1996
(73) Patentinhaber: Mepha AG, CH-4147 Aesch (CH)
(72) Erfinder: Ognyanov, Iliya Vassilev, 1124 Sofia (BG); Konakchiev, Angel Nikolov, 1715 Sofia (BG); Hänni, Ralph, 4414 Füllinsdorf (CH)
(74) Vertreter: Zimmermann, Hans, Dr.

(56) Entgegenhaltungen:
- CHEMICAL ABSTRACTS, vol. 119, no. 23, 6.Dezember 1993 Columbus, Ohio, US; abstract no. 249761a, CHAU ET AT: "Semisynthesis of an antimalarial artesunate" Seite 961; Spalte 2; XP002007034 & TAP CHI DUOC HOC, Bd. 5, 1992, Seiten 10-12,
- CHEMICAL ABSTRACTS, vol. 107, no. 19, 9.November 1987 Columbus, Ohio, US; abstract no. 168241f, LIN ET AL: "Antimalarial activity of new water-soluble dihydroartemisinin derivatives" Seite 13; Spalte 2; XP002007035 & J.MED.CHEM., Bd. 30, Nr. 11, November 1987, Seiten 2147-2150,
- CHEMICAL ABSTRACTS, vol. 98, no. 1, 3.Januar 1983 Columbus, Ohio, US; abstract no. 4420h, LI ET AL: "Studies on anaologs of arteannuin" Seite 395; Spalte 2; XP002007036 & HUAXUE XUEEBAO, Bd. 40, Nr. 6, 1982, Seiten 557-561,
- ACTA CHIM SINICA, vol.40, no.6, 1982, page 557 - 561, ''
- BULL.CHIN.MATERIA.MEDICA, vol.6, no.4, 1981, page 25 - 27, ''
- ACTA PHARM. SIN., vol.16, no.6, 1981, page 429 - 439, ''

## Beschreibung

Die vorliegende Erfindung betrifft ein verbessertes Verfahren zur Herstellung des Dihydroartemisinin-hemisuccinates, mit der chemischen Bezeichnung [3R-(3α,5aβ,-6β, 8aβ, 9α, 10β, 12β, 12aR*)] -Butandisäure-mono(decahydro-3,6,9-trimethyl-3,12-epoxy-12H-pyrano[4,3-j]-1,2-benzodioxepin-10-yl)ester, durch Acylierung von [3R-(3α,5aβ,-6β, 8aβ, 9α, 10, 12β, 12aR*)]-decahydro-10-hydroxy-3,6,9-trimethyl-3,12-epoxy-12H-pyrano[4,3-j]-1,2-benzodioxepin. Mischungen der 10α-Hydroxy- und 10β-Hydroxy-Epimere des letzteren sind auch unter den Namen Dihydroartemisinin und Dihydroqinghaosu bekannt ist. Für das Hemisuccinat und dessen Natriumsalz ist auch die Bezeichnung Artesunate gebräuchlich (und für das Hemisuccinat auch die englische Bezeichnung Artesunic acid).

Das Sesquiterpen-Endoperoxid-Lacton Artemisinin und dessen 10α-Derivat Dihydroartemisinin-hemisuccinat werden in der Chemotherapie gegen gewöhnliche und schwere (akute) Infektionen mit Plasmodium falciparum verwendet, das für 85% der Malariaerkrankungen verantwortlich ist. Die Chemie und die antiprotozoale Wirkung dieser Verbindungen ist in den folgenden Publikationen beschrieben: H.J. Woerdenbag et al., Progress in the Research of Artemisinin-related Antimalarials: An Update, Pharm. World Sci. 16 (4), 169-180 (1994); T.T. Hien et al., Qinghaosu, The Lancet 341, 603-608 (1993); A. R. Butler et al., Artemisinin (Qinghaosu): A New Type of Antimalarial Drug, Chem. Soc. Reviews 85-90 (1992); S.S. Zaman et al., Some Aspects of the Chemistry and Biological Activity of Artemisinin and Related Antimalarials, Heterocycles 32 (8), 1593-1638 (1991); H.J. Woerdenbag et al., Artemisia annua L.: a Source of Novel Antimalarial Drugs, Pharm. Weekblad Sci. 12 (5), 169-181 (1990); D.L. Klayman, Qinghaosu (Artemisinin): An Antimalarial Drug from China, Science 228, 1049-1055 (1985).

Das wasserunlösliche Dihydroartemisinin-hemisuccinat wird üblicherweise oral in Form von Tabletten oder rektal in Form von Suppositorien verabreicht, während das wasserlösliche Natriumsalz (Natriumartesunate) intravenös verabreicht wird.

Dihydroartemisinin-hemisuccinat, zusammen mit einer Reihe weiterer 10-Ester- und 10-Etherderivate von Dihydroartemisinin, wurde von chinesischen Wissenschaftlern Ende 1979 bis Anfang 1980 erstmals synthetisch hergestellt. D. Shaofeng et al.: ³H Labeling of QHS Derivatives, Bull. Chin. Materia Medica 6 (4), 25-27 (1981) und Y. Li et al., Synthesis of Ethers, Carboxylic Esters and Carbonates of Dihydroartemisinin, Acta Pharm. Sin. 16 (6), 429-439 (1981) beschreiben die Herstellung von Dihydroartemisinin-hemisuccinat durch Acylierung von Dihydroartemisinin mit Bernsteinsäureanhydrid in Pyridin. In der letztgenannten Publikation, in der dieses Verfahren als allgemeine Methode A₁ zur Herstellung verschiedener Dihydroartemisinin-10-ester vorgestellt wurde, konnte das Dihydroartemisinin-hemisuccinat mittels Erwärmen von Dihydroartemisinin und Bernsteinsäureanhydrid in Pyridin auf 30°C während 24 Stunden in einer Ausbeute von 60% erhalten werden.

Eine als Methode B₁ bezeichnete, verbesserte Version der Acylierung von Dihydroartemisinin wurde von L. Ying et al. in The Synthesis of Some Carboxylic Esters and Carbonates of Dihydroartemisinin by Using 4-(N,N-Dimethylamino)pyridine as an Active Acylation Catalyst, Acta Chim. Sinica 40 (6), 557-561 (1982) vorgeschlagen und anhand der Herstellung des Dihydroartemisinin-10-valerates im Detail beschrieben. Hierbei wurden 2 mMol Dihydroartemisinin in 30 ml 1,2-Dichlorethan gelöst, mit 4 mMol Valeriansäureanhydrid, 0,33 mMol 4-(N,N-Dimethylamino)pyridin und 29 mMol Triethylamin versetzt, und das Gemisch bei Raumtemperatur gerührt, bis das Dihydroartemisinin aufgebraucht war. Anschliessend wurde das Reaktionsgemisch mit verdünnter Salzsäure angesäuert und die wässrige Phase abgetrennt. Der nach dem Waschen und Trocknen der organischen Phase und dem Abdestillieren des Lösungsmittels erhaltene ölige Rückstand wurde auf Kieselgel unter Verwendung von Petrolether 60-90°/Ethylacetat 10:1 als Eluens chromatographisch gereinigt.

Die Anwendung dieser Arbeitsvorschrift zur Herstellung des Dihydroartemisinin-hemisuccinates, aber unter Verwendung von Dihydroartemisinin, Bernsteinsäureanhydrid und 4-(N,N-Dimethylamino)pyridin im Molverhältnis 1:1,5:0,20 und einer Reaktionszeit von 5 Stunden, ergab das Dihydroartemisinin-hemisuccinat in einer Ausbeute von 65%.

Die bekannten Methoden A₁ und B₁ zur Acylierung von Dihydroartemisinin erfordern die Anwesenheit organischer Basen, nämlich die Anwesenheit von Pyridin in Methode A₁ und die Anwesenheit von Triethylamin und 4-(N,N-Dimethylamino)pyridin in Methode B₁. Die Verwendung organischer Basen bei der Acylierung von Alkoholen mit Acylhalogeniden oder Acylanhydriden entspricht üblicher Praxis, und es ist bekannt, dass organische Basen nicht nur als Katalysatoren, sondern auch zur Neutralisierung der bei der Acylierung freigesetzten Säure dienen können (vgl. F.A. Carey und R.J. Sundberg, Advanced Organic Chemistry, 3. Auflage, Teil A, Seite 476, Plenum Press, New York, 1991).

Neben Chemical Abstracts 119: 249761, wo die Reduktion von Artemisinin mit NaBH₄ und anschliessende Umsetzung mit Bernsteinsäureanhydrid offenbart ist, sind bisher nur diese beiden chinesischen Methoden A₁ und B₁ zur Synthese des Dihydroartemisinin-hemisuccinates bekannt geworden, die jedoch zur Herstellung grösserer Mengen ungeeignet sind und nicht nur wenig ökonomisch sind, sondern auch erhebliche technische und/oder Umweltprobleme hinsichtlich der Aufarbeitung und Entsorgung der Lösungsmittel, Reagentien und Nebenprodukte mit sich bringen würden. Insbesondere ist eine chromatographische Trennung bei der Produktion grösserer Mengen nicht praktikabel. Ferner wird in der verbesserten Version B₁ zwar die Verwendung von Pyridin als Lösungsmittel vermieden, aber 4-(N,N-Dimethylamino)pyridin als Katalysator eingesetzt und als Lösungsmittel 1,2-Dichlorethan verwendet, welches ökologisch nicht ganz unbedenklich ist und nach der Destillation teilweise als Verunreinigung des Produktes zurückbleibt. Im vorbekannten Verfahren B₁ werden zudem vergleichsweise grosse Mengen an Lösungsmittel und Reagentien benötigt, nämlich 15 1 1,2-Dichlorethan und 14 Mol Triethylamin pro Mol Dihydroartemisinin und ferner 50-100% Überschuss an Anhydrid.

Aufgabe der vorliegenden Erfindung ist die Entwicklung eines verbesserten Verfahrens, welches die Nachteile der vorbekannten Verfahren vermeidet und welches insbesondere auch zur Herstellung grösserer Mengen Dihydroartemisinin-hemisuccinat geeignet ist.

Die Aufgabe wird erfindungsgemäss gelöst mit einem Verfahren zur Herstellung des 10α-Epimers von Dihydroartemisinin-hemisuccinat der Formel 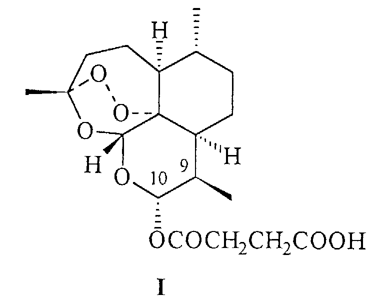 durch Acylierung von Dihydroartemisinin der Formel 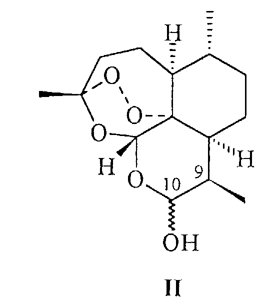 mit Bernsteinsäureanhydrid, welches dadurch gekennzeichnet ist, dass die Acylierung mit 1,0 bis 1,3 Moläquivalenten Bernsteinsäureanhydrid in Gegenwart von 0,5 bis 1,5 Moläquivalenten Tri(C₁-C₃-alkyl)amin, bezogen auf Dihydroartemisinin, in einem niedrig siedenden, neutralen, wassermischbaren, inerten organischen Lösungsmittel oder Lösungsmittelgemisch erfolgt und anschliessend das Hemisuccinat bei pH 5 bis 8 isoliert wird.

Das erfindungsgemässe Verfahren vermeidet die Verwendung von Pyridin oder 1,2-Dichlorethan, und es gestattet zugleich, auf 4-(N,N-Dimethylamino)pyridin als Katalysator völlig zu verzichten. Überraschenderweise wurde ferner gefunden, dass die Lösungsmittel- und Reagentienmengen deutlich vermindert werden können und dennoch eine erhebliche Verbesserung der Ausbeute auf Werte von etwa 93% und höher resultiert. Zudem läuft die erfindungsgemässe Acylierungsreaktion wesentlich rascher ab als die vorbekannten Reaktionen, und das Produkt kann direkt in kristalliner Form in einer Reinheit von über 98% erhalten werden, womit sich chromatographische Trennverfahren erübrigen. Das erfindungsgemässe Verfahren eignet sich somit vorzüglich zur Herstellung von Dihydroartemisinin-hemisuccinat, auch im industriellen Massstab, und es besitzt gegenüber den vorbekannten Verfahren erhebliche Vorteile in ökonomischer und ökologischer Hinsicht.

Das erfindungsgemässe Verfahren liefert nur das 10α -Epimer des Dihydroartemisinin-hemisuccinates, unabhängig von der Konfiguration am C₁₀-Atom des Eduktes der Formel II. Als Ausgangsmaterial im erfindungsgemässen Verfahren kann daher das 10α-Hydroxyepimer, das 10β-Hydroxyepimer oder ein Gemisch dieser Epimere verwendet werden.

Die Acylierung von Dihydroartemisinin kann bei völligem Wasserausschluss grundsätzlich mit einer äquimolaren Menge an Bernsteinsäureanhydrid durchgeführt werden. Im allgemeinen ist jedoch die Verwendung eines geringen Überschusses zu empfehlen.

Als Base eignen sich grundsätzlich alle Tri(C₁-C₃-alkyl)amine, wie Trimethylamin, Methyldiethylamin, Triethylamin und Tripropylamin oder deren Gemische. Triethylamin ist im allgemeinen bevorzugt. Das Tri(C₁-C₃-alkyl)amin wird zweckmässigerweise in einer Menge von mindestens 0,5 Moläquivalenten, bezogen auf Dihydroartemisinin, verwendet, um eine ausreichende Pufferung des Reaktionsmediums zu gewährleisten. Bevorzugt ist eine Menge von 0,7 bis 1,2 Moläquivalenten, beispielsweise 0,8 Moläquivalente.

Die Acylierung erfolgt erfindungsgemäss in einem niedrig siedenden, neutralen, wassermischbaren, inerten organischen Lösungsmittel oder einem Gemisch solcher Lösungsmittel. Die Ausdrücke "niedrig siedend" und "neutral" bezeichnen im Rahmen der vorliegenden Erfindung solche Lösungsmittel, die einen niedrigen Siedepunkt von vorzugsweise höchstens etwa 120°C und keine sauren oder basischen Gruppen (wie Carboxylgruppen, Aminogruppen etc.) aufweisen. Vorzugsweise beträgt jedoch der Siedepunkt des Lösungsmittels mindestens etwa 50°C, wenn die Acylierung nicht in einem geschlossenen Reaktor unter Druck durchgeführt wird. Offenkettige oder cyclische Ketone und Ether, wie Aceton, Methylethylketon, Tetrahydrofuran, Dioxan und dergleichen, sind im allgemeinen bevorzugt. Besonders bevorzugt sind Aceton, Tetrahydrofuran, Dioxan und Gemische dieser Lösungsmittel. Im allgemeinen sind etwa 3-5 l Lösungsmittel pro 1 kg Dihydroartemisinin zur Durchführung der Acylierung völlig ausreichend. Die Lösungsmittelmenge ist jedoch nicht kritisch, und insbesondere können auch höhere Mengen verwendet werden.

Temperatur und Druck der Acylierungsreaktion sind nicht kritisch. Im allgemeinen ist jedoch eine Temperatur von 20 bis 60°C bevorzugt. Insbesondere kann die Reaktion auch bei Atmosphärendruck und Raumtemperatur durchgeführt werden. Die Reaktionsdauer beträgt unter diesen Bedingungen typischerweise etwa 0,5 Stunden. Es ist jedoch zu empfehlen, den Reaktionsverlauf zum Beispiel mittels Dünnschichtchromatographie zu überwachen.

Nach beendeter Acylierung wird mittels Säure ein pH-Wert von 5-8 eingestellt und das gewünschte Dihydroartemisinin-hemisuccinat isoliert. Hierbei wird mit Vorteil zuerst Wasser und dann Säure zugesetzt, um das Produkt in kristalliner Form auszufallen und die Abtrennung des Produktes durch Filtration zu erleichtern. Vorzugsweise werden etwa 3-12 l Wasser pro 1 kg Dihydroartemisinin verwendet. Zur Neutralisierung eignen sich organische und anorganische Säuren, wie Essigsäure, Salzsäure, Schwefelsäure und dergleichen; bevorzugt ist verdünnte Salzsäure. Vorzugsweise wird ein pH-Wert von 5,5-7,5, insbesondere etwa 6,5, eingestellt. Die Temperatur ist nicht kritisch und kann beispielsweise 20 bis 70°C betragen. Insbesondere kann die Aufarbeitung auch bei Raumtemperatur oder tieferer Temperatur erfolgen.

Die Trocknung des erhaltenen Hemisuccinates erfolgt vorzugsweise bei einer Temperatur von höchstens 60°C.

Das als Ausgangsmaterial verwendete Dihydroartemisinin kann in bekannter Weise durch Reduktion von Artemisinin mit Natriumborhydrid hergestellt werden; vgl. A. Brossi et al., Arteether, a New Antimalarial Drug: Synthesis and Antimalarial Properties, J. Med. Chem. 31, 645-650 (1988).

Das erfindungsgemässe Verfahren wird durch die folgenden Beispiele weiter veranschaulicht. Als Ausgangsmaterial wurde jeweils ein Gemisch des 10α- und des 10β-Epimers von Dihydroartemisinin im ungefähren Verhältnis von 40:60 verwendet, welches weniger als 1% nicht reduziertes Artemisinin enthielt. Die Reagentien und Lösungmittel entsprachen der Merck-Qualität "pure". Die Reinheit des erhaltenen Dihydroartemisinin-10α-hemisuccinates wurde mittels Dünnschichtchromatographie, Schmelzpunktmessung und HPLC kontrolliert, und dessen Identität durch Vergleich mit einer authentischen Probe mittels ¹H-NMR- und IR-Spektren, Dünnschichtchromatographie und HPLC bestätigt.

### Beispiel 1

0,45 g (4,5 mMol) Bernsteinsäureanhydrid wurden bei 25°C in einem Gemisch von 3 ml trockenem Aceton und 0,4 ml (0,29 g; 2,9 mMol) Triethylamin gelöst. Die Lösung wurde mit 1,0 g (3,52 mMol) Dihydroartemisinin versetzt und das Gemisch gerührt bis zur vollständigen Umsetzung des Dihydroartemisinin (Kontrolle mittels Dünnschichtchromatographie). Anschliessend wurde das Reaktionsgemisch unter Rühren mit 12 ml Wasser versetzt, und die halbkristalline, milchige Suspension mit verdünnter Salzsäure auf pH 6,5 gestellt. Der erhaltene, feste, kristalline Niederschlag wurde abfiltriert und mit destilliertem Wasser gewaschen, bis das Filtrat neutral und frei von Chloridionen war. Nach der Trocknung wurden 1,28 g (95,0%) gemäss Dünnschichtchromatogramm reinem Dihydroartemisinin-hemisuccinat mit Smp. 140-142°C erhalten.

### Beispiel 2

Mit 1,0 g Dihydroartemisinin und unter Durchführung wie in Beispiel 1 beschrieben, aber mit 4 ml Tetrahydrofuran anstelle von Aceton, wurden 1,30 g (96,5%) Dihydroartemisinin-hemisuccinat mit Smp. 139,5-142°C erhalten.

### Beispiel 3

Mit 1,0 g Dihydroartemisinin und unter Durchführung wie in Beispiel 1 beschrieben, aber mit 4 ml Dioxan anstelle von Aceton, wurden 1,25 g (92,8%) Dihydroartemisinin-hemisuccinat mit Smp. 140, 5-142, 5°C erhalten.

### Beispiel 4

Mit 1,0 g Dihydroartemisinin und unter Durchführung wie in Beispiel 1 beschrieben, aber mit 4 ml eines 1:1-Gemisches von Aceton und Tetrahydrofuran anstelle von Aceton, wurden 1,30 g (92,8%) Dihydroartemisinin-hemisuccinat mit Smp. 141-143°C erhalten.

### Beispiele 5-8

Nach der gleichen Methode und unter gleichen Bedingungen wie in Beispiel 1 beschrieben, wurden unter Verwendung von höheren Mengen an Dihydroartemisinin (DHA), Bernsteinsäureanhydrid (SA), Triethylamin [N(C₂H₅)₃] und Aceton vier weitere Versuche durchgeführt. Die verwendeten Mengen, die erhaltenen Ausbeuten an Dihydroartemisinin-hemisuccinat und der Schmelzpunkt des Produktes sind in Tabelle 1 angegeben.

**Tabelle 1**

| Beispiel | DHA | SA | N(C₂H₅)₃ | Aceton | Ausbeute | | Smp. |
|---|---|---|---|---|---|---|---|
| | g | g | ml | ml | g | % | °C |
| 5 | 50 | 23 | 20 | 150 | 61,5 | 91,8 | 140 -142 |
| 6 | 100 | 45 | 40 | 300 | 124,7 | 92,2 | 141,5-143,5 |
| 7 | 300 | 135 | 120 | 900 | 394,0 | 97,2 | 140 -142 |
| 8 | 400 | 180 | 160 | 1200 | 515,0 | 95,2 | 140,5-142,5 |

## Patentansprüche

1. Verfahren zur Herstellung des 10α-Epimers von Dihydroartemisinin-hemisuccinat der Formel 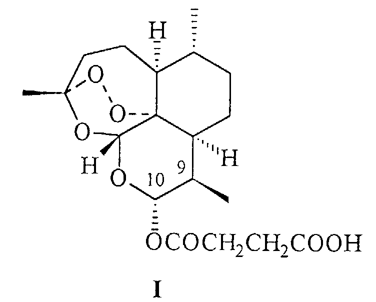 durch Acylierung von Diyhdroartemisinin der Formel 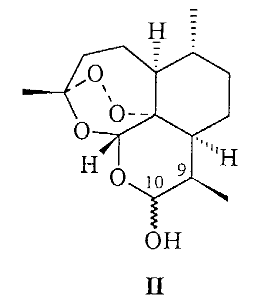 mit Bernsteinsäureanhydrid, dadurch gekennzeichnet, dass die Acylierung mit 1,0 bis 1,3 Moläquivalenten Bernsteinsäureanhydrid in Gegenwart von 0,5 bis 1,5 Moläquivalenten Tri(C₁-C₃-alkyl)amin, bezogen auf Dihydroartemisinin, in einem niedrig siedenden, neutralen, wassermischbaren, inerten organischen Lösungsmittel oder Lösungsmittelgemisch erfolgt und anschliessend das Hemisuccinat bei pH 5 bis 8 isoliert wird.

2. Verfahren nach Anspruch 1, dadurch gekennzeichnet, dass Tri(C₁-C₃-alkyl)amin in einer Menge von 0,7 bis 1,2 Moläquivalenten, bezogen auf Dihydroartemisinin, verwendet wird.

3. Verfahren nach Anspruch 1 oder 2, dadurch gekennzeichnet, dass als Tri(C₁-C₃-alkyl)amin Triethylamin verwendet wird.

4. Verfahren nach einem der Ansprüche 1 bis 3, dadurch gekennzeichnet, dass als organisches Lösungsmittel ein Keton und/oder ein Ether verwendet wird.

5. Verfahren nach einem der Ansprüche 1 bis 4, dadurch gekennzeichnet, dass als organisches Lösungsmittel Aceton, Tetrahydrofuran und/oder Dioxan verwendet wird.

6. Verfahren nach einem der Ansprüche 1 bis 5, dadurch gekennzeichnet, dass das organische Lösungsmittel oder Lösungsmittelgemisch in einer Menge von 3-5 1 pro 1 kg Dihydroartemisinin verwendet wird.

7. Verfahren nach einem der Ansprüche 1 bis 6, dadurch gekennzeichnet, dass die Acylierung bei 20 bis 60°C durchgeführt wird.

8. Verfahren nach einem der Ansprüche 1 bis 7, dadurch gekennzeichnet, dass man das Reaktionsgemisch nach erfolgter Acylierung mit Wasser versetzt und durch Zugabe von Säure einen pH-Wert von 5 bis 8 einstellt.

9. Verfahren nach Anspruch 8, dadurch gekennzeichnet, dass man als Säure verdünnte Salzsäure verwendet.

## Claims

1. Process for the preparation of the 10α epimer of dihydroartemisinin hemisuccinate of the formula 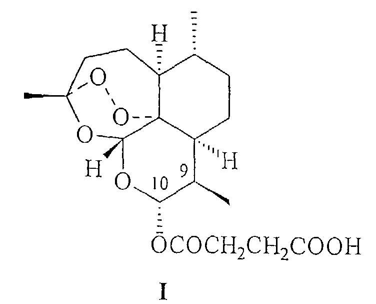 by acylation of dihydroartemisinin of the formula 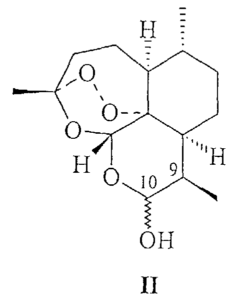 with succinic anhydride, characterized in that the acylation is carried out with 1.0 to 1.3 molar equivalents of succinic anhydride in the presence of 0.5 to 1.5 molar equivalents of tri(C₁-C₃-alkyl)amine, relative to dihydroartemisinin, in a low-boiling, neutral, water-miscible, inert organic solvent or solvent mixture and the hemisuccinate is then isolated at pH 5 to 8.

2. Process according to Claim 1, characterized in that tri(C₁-C₃-alkyl)amine is used in an amount of 0.7 to 1.2 molar equivalents, relative to dihydroartemisinin.

3. Process according to Claim 1 or 2, characterized in that the tri(C₁-C₃-alkyl)amine used is triethylamine.

4. Process according to one of Claims 1 to 3, characterized in that the organic solvent used is a ketone and/or an ether.

5. Process according to one of Claims 1 to 4, characterized in that the organic solvent used is acetone, tetrahydrofuran and/or dioxane.

6. Process according to one of Claims 1 to 5, characterized in that the organic solvent or solvent mixture is used in an amount of 3-5 l per 1 kg of dihydroartemisinin.

7. Process according to one of Claims 1 to 6, characterized in that the acylation is carried out at 20 to 60°C.

8. Process according to one of Claims 1 to 7, characterized in that water is added to the reaction mixture after acylation has taken place and a pH of 5 to 8 is set by addition of acid.

9. Process according to Claim 8, characterized in that the acid used is dilute hydrochloric acid.

## Revendications

1. Procédé de préparation de l'épimère 10α de l'hémisuccinate de dihydroartémisinine répondant à la formule 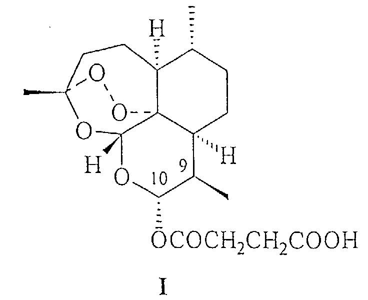 par acylation de la dihydroartémisinine de formule 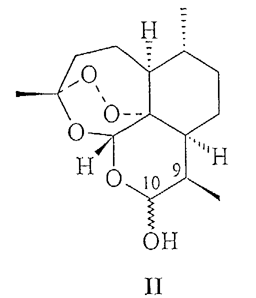 au moyen d'anhydride succinique, caractérisé en ce que l'acylation est effectuée avec de 1,0 à 1,3 équivalents molaires d'anhydride succinique en présence de 0,5 à 1,5 équivalents molaires de tri(alcoyle en C₁-C₃)amine, calculés par rapport à la dihydroartémisinine, dans un solvant organique inerte, miscible à l'eau, neutre et de bas point d'ébullition ou dans un mélange de tels solvants, et que l'hémisuccinate est ensuite isolé à un pH de 5 à 8.

2. Procédé selon la revendication 1, caractérisé en ce que la tri(alcoyle en C₁-C₃)amine est utilisée en une quantité de 0,7 à 1,2 équivalents molaires, calculés par rapport à la dihydroartémisinine.

3. Procédé selon la revendication 1 ou 2, caractérisé en ce que la tri(alcoyle en C₁-C₃)amine utilisée est la triéthylamine.

4. Procédé selon l'une quelconque des revendications 1 à 3, caractérisé en ce que le solvant organique utilisé est une cétone ou un éther.

5. Procédé selon l'une quelconque des revendications 1 à 4, caractérisé en ce que le solvant organique utilisé est l'acétone, le tétrahydrofurane et/ou le dioxanne.

6. Procédé selon l'une quelconque des revendications 1 à 5, caractérisé en ce que le solvant organique ou le mélange de solvants organiques est utilisé en une quantité de 3 à 5 litres par kg de dihydroartémisinine.

7. Procédé selon l'une quelconque des revendications 1 à 6, caractérisé en ce que l'acylation est effectuée de 20 à 60 °C.

8. Procédé selon l'une quelconque des revendications 1 à 7, caractérisé en ce que le mélange réactionnel est traité à l'eau après l'acylation et qu'il est ajusté à un pH de 5 à 8 par adjonction d'acide.

9. Procédé selon la revendication 8, caractérisé en ce que l'acide utilisé est l'acide chlorhydrique dilué.
